# EUROPEAN PATENT SPECIFICATION

(11) **EP 1 431 274 B1**
(45) Date of publication and mention of the grant of the patent: **15.08.2007**
(21) Application number: 03029005.0
(22) Date of filing: 16.12.2003
(51) Int. Cl.: C07C 213/08, C07C 231/12, C07C 227/14, C07C 215/76, C07C 215/68, C07C 237/40, C07C 229/60, C07C 229/56

(54) **Synthesis of mono-N-substituted functionalized anilines**
Synthese von Mono-N-substituierten funktionalisierten Anilinen
Synthése d'anilines fontionnalisées mono-N-substituées

(30) Priority: 18.12.2002 IT PD20020325
(43) Date of publication of application: 23.06.2004
(73) Proprietor: Consorzio Interuniversitario Nazionale "La Chimica Per L'Ambiente", 30123 VENEZIA (IT)
(72) Inventor: Selva, Maurizio, 30123 Venezia (IT); Tundo, Pietro, 30123 Venezia (IT)
(74) Representative: Modiano, Micaela Nadia

(56) References cited:
- US-A- 4 940 817
- MAURIZIO SELVA ET AL.: "Selective mono-N-methylation of primary aromatic amines by dimethyl carbonate over faujasite X- and Y-type zeolites" J. CHEM. SOC., PERKIN TRANS. 1,, 1997, pages 1041-1045, XP009028734
- MAURIZIO SELVA ET AL.: "Reaction of Primary Aromatic Amines with Alkyl Carbonates over NaY Faujasite: A Convenient and Selective Acces to Mono-N-Alkyl Anilines" J. ORG. CHEM., vol. 66, no. 3, - 2001 pages 677-680, XP002276884
- MAURIZIO SELVA ET AL : "Mono-N-methylation of Primary Amines with Alkyl Methyl Carbonates over Y Faujasites.2. Kinetics and Selectivity" J. ORG. CHEM., vol. 67, no. 26, 2002, pages 9238-9247, XP002276885
- P. R. HARI PRASAD RAO ET AL.: "Selectivity to N-mono or dialkylation in the reaction of aniline with dimethyl carbonate on faujasite, EMT and beta alkyline zeolites" CATALYSIS LETTERS , vol. 31, 1995, pages 115-120, XP009028752
- ZI-HUA FU ET AL.: "Selective N-monomethylation of aniline with dimethyl carbonate over Y-zeolites" CATALYSIS LETTERS, vol. 22, 1993, pages 277-281, XP009028753
- BAO LIAN SU ET AL.: "Alkylation of aniline with methanol: Change in selectivity with acido-basicity of faujasite catalysts " APPLIED CATALYSIS A: GENERAL 124, - 1995 pages 73-80, XP002276887

## Description

The present invention relates to the synthesis of anilines mono-N-substituted and variously functionalized on the aromatic core.

Preparation of secondary aromatic amines has a primary role in many synthesis processes.

In particular, compounds that are the result of the introduction of alkyl, alkenyl and benzyl radicals on the aniline nitrogen atom are known for their importance in the synthesis of pharmaceutical components and in the color industry.

These products are usually obtained by direct reaction of primary anilines with alkyl halides or dialkylsulfates and allyl and benzyl halides, which are well-known in the chemical field for this application.

However, there have been cases in which this process entailed considerable difficulties.

In particular, reference is made here to the case in which the initial anilines were already functionalized (for example directly on the benzene core) with chemical groups such as hydroxyl, carboxyl and carboxyamine, which are potentially able to interfere due to their reactivities.

For example, direct alkylation of molecules such as aminophenols, aminobenzyl alcohols, aminobenzoic acids and aminobenzamides led to the unintended production of high percentages of N,N-dialkylated product (see in this regard i) Gibson, M.S.; The chemistry of the amino group 1968, Chapter 2, pages 45-62; ii) WO 9112000, and iii) Kalgutkar et al. J. Med. Chem. 1998, 41, 4800), and even to nonselective alkylation of other potentially interfering atoms.

In this regard, an alkylation that affected the molecules cited above, characterized by the presence of nucleophilic oxygen, would have led to N,O-dialkylated byproducts in final percentages that differed according to the reaction conditions used. (Yun, H. S. Chem. Abstr. 1974, 83, 42952).

In addition to reducing the overall yield of the intended product, this requires long and expensive purification procedures.

Maurizio Selva et al. "Selective mono-N-methylation of primary aromatic amines by dimethyl carbonate over faujasite X- and Y-type zeolites", J. CHEM. SOC. Perkin Trans. 1, 1997, pages 1041-1045, investigates the reaction of dimethyl carbonate (DMC) with different primary aromatic amines under batch conditions (autoclave) in the presence of Y- and X-type zeolites. In particular, the substituted anilines which have been investigated are p-N02 aniline, p-CN aniline, o-CH3O2C aniline and 2,6-dimethyl aniline. No mention is made to reactions involving an aniline substrate substituted with electron rich substituents.

Maurizio Selva et al. "Reaction of primary aromatic amines with alkyl carbonates over NaY faujasite: a convenient and selective access to mono-N-alkyl anilines", J. ORG. CHEM., vol. 66, no. 3, 2001 pages 677-680, discloses that at atmospheric pressure and at 130-160°C, primary aromatic amines (p-XC6H4NH2, X = H, Cl, NO2) are mono-N-alkylated in a single step, with symmetrical and asymmetrical dialkyl carbonates in the presence of a commercially available NaY faujasite. No mention is made to reactions involving a substrate whose substituents can interfere with the alkylation of the nitrogen atom.

Maurizio Selva et al. "Mono-N-methylation of primary amines with alkyl methyl carbonates over faujasites.2. Kinetics and selectivity", J. ORG. CHEM., vol. 67, no. 26, 2002 pages 9238-9247, discloses the author's efforts in reporting some of the general conclusions drawn from the analysis of the kinetics of the monomethylation reaction of different primary amines carried out by means of the asymmetric carbonate 2-(2-methoxyethoxy)methylethyl carbonate in the presence of several Y-type zeolites. This document does not relate to reactions that involve anilines whose substituents can compete with the nitrogen atom for the alkylation.

P.R. Hari Prasad Rao et al. "Selectivity to N-mono or dialkylation in the reaction of aniline with dimethyl carbonate on faujasite, EMT and beta alkyline zeolites", Catalysis Letters, vol. 31, 1995, pages 115-120, discloses the result obtained with some zeolitic systems in the alkylation of non-substituted aniline. No mention is made to reactions involving a substrate whose substituents can interfere with the alkylation of the nitrogen atom.

Zi-Hua Fu et al. "Selective N-monomethylation of aniline with dimethyl carbonate over Y-zeolites", Catalysis Letters, vol. 22, 1993, pages 277-281, discloses the methylation of non-substituted aniline with dimethyl carbonate. Even though the zeolites tested are, inter alia, of Y and X type, exchanged with alkaline cations, this document exclusively focuses on the alkylation of non-substituted aniline, while no mention is made to the alkylation of substituted anilines, in particular of anilines substituted with nucleophile groups.

Bao Lian Su et al. "Alkylation of aniline with methanol: change in selectivity with acido-basicity of faujasite catalysts", Applied Catalysis A: general 124, 1995, pages 73-80, exclusively relates to the alkylation of non-substituted aniline with methanol. No mention is made to the investigation of the alkylation of substituted anilines, in particular of anilines substituted with nucleophile groups.

US 4,940,817 discloses a process for the preparation of N-alkylanilines, in which anilines which are not substituted on the N atom are reacted with N,N-dialkylanilines in the presence of zeolites at elevated temperature. In particular, it relates to a transalkylation reaction between a N,N-dialkylaniline and a not N-substituted aniline which can be substituted on the aromatic ring with R2 and R3 where none of these substituents can compete with the nitrogen atom for the alkylation thus leading to a poor chemoselectivity.

In order to minimize the incidence of these parasitic reactions, alternative synthetic approaches have been found which are based, for example, on the reductive amination of anilines by using formaldehyde or methyl esters (Krishnamurthy, S. Tetrahedron Letts. 1982, 3315), on the hydrolysis of benzoxazol derivatives (Sakurai, T.; Yamada, S.; Inoue, H. Bull. Chem. Soc. Jpn. 1986, 59, 2666), or on methods for selective O-dealkylation (Guo, Z.; Ramirez, J.; Li, J.; Wang, P. G. J. Am. Chem. Soc. 1998, 120, 3726).

These indirect methods usually entail lower yields than equivalent direct processes and, most of all, entail reaction conditions (particularly temperature and pressure) that are often drastic and incompatible with any labile functional groups.

Finally, despite the efforts made also in terms of obtaining the raw materials (which is not always easy), the problem of N,N-dialkylation still has not been solved (see in this regard EP-A-0855386, JP 52071424 and JP 2000095740).

Secondly, one should also remember that over the last decade the problem of possible environmental impact has become a decisive factor in assessing the applicability of new industrial processes and for any change to existing processes.

In this regard, the alkylating agents conventionally used (such as the already-mentioned alkyl halides and dialkylsulfates, and benzyl and allyl halides) are unfortunately known also for their considerable toxicity and for the particular conditions required in order to use them.

### SUMMARY OF THE INVENTION

The aim of the present invention is to provide a general process that allows to prepare the corresponding secondary aromatic amine by selective introduction of an alkyl, allyl or benzyl group at the nitrogen atom of a primary aniline functionalized at the aromatic ring.

Within this aim, an object of the present invention is to provide a direct synthesis process that leads to the mono-N-substitution of functionalized anilines, avoiding products of N,N-disubstitution (dialkyl, diallyl, or dibenzyl derivatives).

Another object of the present invention is to provide a direct and chemoselective process for mono-N-substitution at the nitrogen atom of functionalized anilines with groups that are potentially capable of interfering with said process.

A further object of the present invention is to provide a direct and selective synthesis method for mono-N-substitution at the nitrogen atom of functionalized anilines that is environmentally compatible as a whole.

This aim and these and other objects that will become better apparent hereinafter are achieved by a process for direct synthesis of mono-N-substituted anilines having the general formula (I) 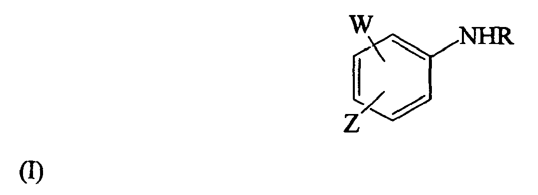 wherein
R indicates a linear or branched saturated carbon chain, preferably comprising 1 to 7 carbon atoms, an unsaturated carbon chain with the double carbon-carbon bond also in the allyl position (2,3) with respect to the nitrogen atom of the amines (I), comprising 3 to 7 carbon atoms, or a benzyl group or a benzyl group substituted at the aromatic ring with methyl and ethyl radicals;
W is selected from the group consisting of -OH, -CH2OH, -COOH and -CONH2 and can be ortho, meta or para with respect to the carbon atom to which the nitrogen atom is attached;
Z is selected from the group consisting of -H, -halogen, -alkyl, -alkoxy, -N02 and -CN;
said process comprising the step of reacting, in the presence of a solvent, a compound having the general formula (II): 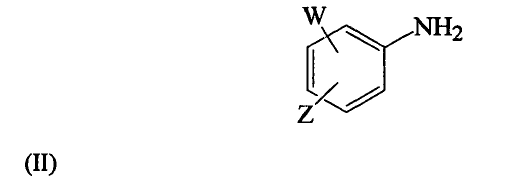 wherein W and Z are as defined above,
with an organic carbonate selected from the group consisting of compounds having the general formula (III) 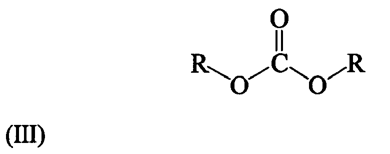 wherein R is as defined above,
and compounds having the general formula (IV) 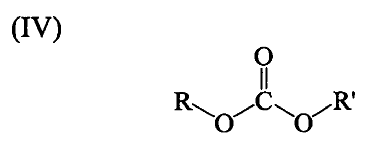 wherein R' is selected from the group consisting of CH3(OCH2CH2)n-with n ≥ 2 and linear or branched alkyl radicals (where said radicals have in particular at least three carbon atoms), in the presence of a faujasite selected from the group consisting of X- faujasite exchanged with alkaline metals and Y- faujasite exchanged with alkaline metals.

Preferred examples of R groups according to the present invention are methyl, ethyl, allyl and benzyl.

Even more preferably, R is methyl and ethyl.

The term "alkyl" used in the context of the present invention designates a linear or branched saturated chain comprising 1 to 7 carbon atoms.

The term "alkoxy" used in the context of the present invention designates a linear or branched saturated chain joined to the aniline core by means of an oxygen bridge.

Preferably, the alkoxy radical comprises 1 to 5 carbon atoms. Preferred examples are methoxy, ethoxy, and propoxy.

According to the present invention, the reaction being considered is performed by placing in contact the aniline substrate, the organic carbonate and the faujasite.

The compounds (II) were made to react with the organic carbonate in the presence of faujasite at a temperature comprised between 70°C and 190°C, preferably comprised between 90°C and 150°C and even more preferably comprised between 70°C and 90°C if dimethyl carbonate is used, between 70°C and 130°C if diethyl carbonate, diallyl carbonate and dibenzyl carbonate are used, and above 130°C if the asymmetric carbonates having the formula (IV) are used.

Moreover, the process provided here allows to achieve the intended aim and objects even at atmospheric pressure, but this does not forbid the possibility to perform the alkylation reaction in an autoclave if required by the temperatures used.

In the specific case of methylation and ethylation reactions, if temperatures higher than the boiling points of the alkylating agents (DMC: 90°C; DEC: 126°C) are required, the reaction can still be performed at atmospheric pressure by using asymmetric carbonates having the general formula (IV): 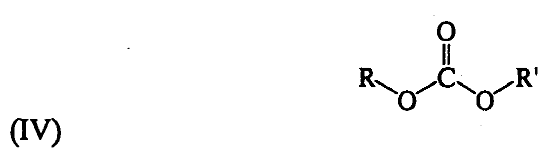 wherein R' is an alkyl chain having 3 or more carbon atoms or an oxymethylene chain such as CH3(OCH2CH2)n- with n a 2.

Oxymethylene derivatives are preferable because they are easier to synthesize and because of their low vapor pressure, which allows to use them over a wider temperature range.

In particular, preferred examples of carbonates having the formula (IV) are 2-(2-methoxyethoxy)ethyl-methylcarbonate (having the formula CH3(OCH2CH2)2OCO2Me) and 2-(2-methoxyethoxy)ethyl-ethylcarbonate (having the formula CH3(OCH2CH2)2OCO2Et).

The temperature preferably used for the carbonates having the formula (IV) is comprised between 130° and 190°C.

Regardless of the temperature and pressure conditions used, it has been found that the reaction is usually completed after a time ranging from 3 to 30 hours.

If the use as substrates of aniline derivatives that can be degraded easily as a consequence of hydrolytic or oxidative phenomena is desired, a modified atmosphere, for example by using inert gases such as nitrogen and argon is advantageously used in the present invention.

Generally, the organic carbonates (III) or (IV) used, if added in excess with respect to the organic substrate (II), are themselves perfectly capable of also acting as solvents.

Therefore, it has been found that the molar ratio of use of said alkylating agents with respect to the amount of aniline substrate can vary effectively between 10:1 and 50:1 and preferably between 25:1 and 35:1.

However, when the substrate has an excessively low coefficient of solubility in these solvents, it is possible to use co-solvents, among which mention is made here of dimethyl ethers derived from glycols and more particularly 1,2-dimethoxyethane (DME) and triglyme (triethylene glycol dimethyl ether).

These optional substances are used in a mixture with the carbonates (III) or (IV) and in ratios carbonate:co-solvent between 1:1 and 1:5 (v/v).

As noted above, the present invention provides a process in which the reaction conditions, particularly as regards the temperature, are particularly mild, and this aspect is a considerable advantage.

The achievement of high yields and excellent chemoselectivity even in these mild conditions has been achieved here mainly by way of the combined use of the carbonates cited above and of faujasites.

This last class of compounds indicates solids of the aluminosilicate type, which due to their structure can contain various types of ions of alkaline metals, alkaline-earth metals and/or transition metals.

Preferably, according to the present invention X-faujasites and Y-faujasites, both exchanged with alkaline metals, and even more preferably Y-faujasites exchanged with sodium, are used as catalysts.

As regards the quantities in which they are used, it has been found that it is possible to vary the percentages considerably while keeping, however, an interval of weight ratio: Y-faujasite / aniline substrate between 1:10 and 3:1 and more preferably between 1:1.5 and 1:1.

Further characteristics and advantages of the process according to the present invention will become better apparent from the following detailed description of some preferred but not exclusive embodiments thereof.

### Examples.

In the performed experimental tests reported here, the quantity of organic substrate (functionalized aniline) was varied over a range comprised between 0.5 g and 10 g, preferably 1g.

Examples 1 to 7 and 12 refer to reactions performed in conventional reaction flasks and at atmospheric pressure, while examples 8 to 11 relate to processes performed in an autoclave.

### Example 1

N-methyl-p-aminophenol.

The p-aminophenol (1 g) is dissolved in a mixture consisting of dimethyl carbonate (hereafter referenced as DMC) (10 ml) and DME (35 ml).

The NaY-faujasite (1 g) is added to the resulting mixture and heating to 90°C is performed.

The reaction, which is monitored by gas chromatography optionally associated with mass spectrometry, is completed after 7 hours, providing a practically quantitative conversion.

At this point the solid catalyst is eliminated by filtration and the monomethylated product can thus be recovered.

Instrumental analysis performed after solvent evaporation yielded a selectivity value of 99% for the N-monomethylated product and a total yield of 91%.

### Example 2

### N-methyl-o-aminophenol

The o-aminophenol (1 g) is dissolved in pure DMC (30 ml).

In this case, no co-solvent is added.

The NaY-faujasite (1 g) is added to the resulting mixture and heating to 90°C is performed.

The reaction, which is monitored by means of gas chromatography optionally associated with mass spectrometry, is completed after 3 hours, providing a practically quantitative conversion.

At this point the solid catalyst is eliminated by filtration and the monomethylated product can thus be recovered.

Instrumental analysis performed after solvent evaporation reveals a total yield of 99% of N-monomethylated product.

### Example 2a

The method of example 2 is repeated, but in this case the quantity of NaY-faujasite is limited to 1/10 of the quantity of o-aminophenol (again 1 g).

The reaction is completed after 38 hours, giving a total yield of 93% of N-monomethylated product.

### Example 3

### N-methyl-m-aminophenol

A mixture of m-aminophenol (1 g) in DMC (30 ml) is prepared according to the method described in example 1 without resorting to co-solvents.

In this case, the NaY-faujasite is added in a quantity that is equal in weight with respect to the m-aminophenol.

The reaction is performed for 7 hours.

The yield of N-methyl-m-aminophenol is 89%.

### Example 3a

The reaction of example 3 is reproduced, again starting from m-aminophenol (1 g), but in this case the quantity of NaY-faujasite is 1/10 with respect to the aromatic substrate.

The time required for the reaction to complete is 42 hours, but the final yield still remains high and is more precisely equal to 92%.

### Example 4

### N-methyl-p-aminobenzyl alcohol

The p-aminobenzyl alcohol (1 g) is dissolved in pure DMC (30 ml).

The NaY-faujasite (1 g) is added to the resulting mixture and heating to 90°C is performed.

The reaction, which is monitored by gas chromatography optionally associated with mass spectrometry, is completed after 8 hours.

The mono-N-methylation selectivity is 94%.

The solid catalyst (NaY-faujasite) is again eliminated by filtration.

The product is recovered after purification performed with flash chromatography (eluent: ethyl acetate/petroleum ether, 1:4 v/v).

The final yield on N-methyl-p-aminobenzyl alcohol is equal to 77%.

### Example 5

### N-methyl-o-aminobenzyl alcohol

A mixture of o-aminobenzyl alcohol (1 g) in DMC (30 ml) is prepared according to the method described in example 1.

The two compounds are made to react in the presence of NaY-faujasite (1 g) for 12 hours in the conditions described in the preceding examples.

The yield on the isolated title product is 92%.

The mono-N-methylation selectivity is 99%.

### Example 6

### N-methyl-p-aminobenzamide

According to the method described in example 1, the p-aminobenzamide (1 g) is made to react with the DMC (50 ml), which also acts as a solvent.

After 24 hours, the reaction is completed, with 93% mono-N-methylation selectivity.

The methylated product is recovered by flash column chromatography (eluent: ethyl acetate/petroleum ether, 1:4 v/v) and the final yield is 86%.

### Example 7

### N-methyl-o-aminobenzamide

The protocol of example 6 is now repeated, except that the quantity of DMC is slightly reduced (30 ml).

The reaction is extended for 22 hours, leading to a yield after purification of 91%.

### Example 8

### N-methyl-p-aminobenzoic acid

A solution of p-aminobenzoic acid (1 g) in DMC (30 ml) is prepared and the NaY-faujasite according to the protocol of example 1 is then added.

However, differently from the preceding examples, in this case the temperature to which the mixture is brought is 130°C and therefore the reaction must be performed in an autoclave (it is noted in this regard that the boiling point of DMC is 90°C).

After 9 hours, the raw reaction product is recovered and purified by flash column chromatography (eluent: ethyl acetate/petroleum ether, 1:4 v/v).

The yield of mono-N-methylation on the isolated product is 74%.

### Example 9

### N-methyl-o-aminobenzoic acid

The protocol of example 8 is repeated, except that the temperature at which the reaction is performed is 150°C.

After 5 hours, the product is recovered, obtaining a yield equal to 83%.

### Example 10

### Ethyl N-methyl-o-aminobenzoate

In the same manner as in example 8, the ethyl o-aminobenzoate (1 g) is made to react in an autoclave at 150°C with the DMC (30 ml) and, obviously, in the presence of the solid catalyst.

After 8 hours, analysis of a sample by gas chromatography confirms that the only product of the reaction is ethyl N-methyl-o-aminobenzoate (65% yield calculated on the chromatogram), while there are no traces of the product derived from the possible transesterification of the ethyl with the methyl (which would have yielded methyl N-methyl-o-aminobenzoate).

### Example 11

### Methyl N-ethyl-o-aminobenzoate

The alkylation reaction of the preceding example is reproduced here, but the DMC is replaced with diethyl carbonate (30 ml), while the substrate remains o-aminobenzoate (1 g).

After 8 hours, analysis of a sample by gas chromatography confirms that the only product of the reaction is methyl N-ethyl-o-aminobenzoate (23% yield at the time of analysis calculated on the chromatogram), while there are no traces of the product that derives from the possible transesterification of the ethyl with the methyl (which would have yielded ethyl N-ethyl-o-aminobenzoate).

### Example 12

### N-methyl-o-aminobenzoic acid

The o-aminobenzoic acid (1 g) is dissolved in a mixture constituted by triethylene glycol dimethyl ether (triglyme, 15 ml) and 2-(2-methoxyethoxy)ethyl-methylcarbonate (CH3(OCH2CH2)2OCO2CH3, 7 ml).

The NaY-faujasite (1 g) is added to the mixture and heating to 150°C is performed at atmospheric pressure, in the presence of inert gas.

After 12 hours, the reaction is complete: gaschromatographic analysis indicates the product of mono-N-methylation at 93%.

### Example 13

### N-ethyl-o-aminobenzoic acid

The o-aminobenzoic acid (1 g) is dissolved in a mixture constituted by triethylene glycol dimethyl ether (triglyme, 15 ml) and 2-(2-methoxyethoxy)ethyl-ethylcarbonate [Me(OCH2CH2)2OCO2Et, 7 ml].

The NaY-faujasite (1 g) is added to the mixture and heating is performed to 150°C, at atmospheric pressure, in the presence of inert gas.

After 18 hours, conversion is 87%: gaschromatographic analysis indicates a mono-N-ethylation selectivity of 97%.

One considerably important aspect revealed by the cited examples (in particular by examples 1-8) is that the present invention allows to use organic carbonates while maintaining reaction temperatures that are distinctly lower than those cited in the background art (Trotta, F.; Tundo, P.; Moraglio, G.; J. Org. Chem. 1987, 52, 1300).

The advantages that arise from the adoption of low temperatures are many and are well-known to anyone working in this field.

Among them, mention is made in any case of the possibility to use substrates that are sensitive to heat degradation and most of all to use relatively light organic carbonates (with boiling points generally lower than 130°C) as solvents without necessarily having to resort to autoclaves.

Shieh W.-C. et al. have reported (Shieh, W.-C.; Dell, S.; Repic, O. Org. Lett. 2001, 3, 4279 and J. Org. Chem. 2002, 67, 2188) that DMC can be used effectively as an alkylating agent at low temperatures only in sporadic cases and in any case either by adding a non-nucleophilic strong base (such as DBU) as a catalyst or by triggering the reaction with microwaves.

Chemical literature, further, reports that basic or acid catalysts can greatly modify the selectivity of alkylation reactions that use dialkyl carbonates.

The following example is provided as a confirmation of what is described in the background art.

### Example 14 (for comparison)

p-Aminophenol (1 g) is dissolved in a mixture constituted by dimethyl carbonate (10 ml) and dimethyl formamide (15 ml).

Potassium carbonate (2 equivalents for each equivalent of p-aminophenol) is added to the resulting solution as a catalyst and heating to 125°C is performed.

After 5 hours, instrumental analysis of a sample of the mixture showed that conversion was 77% but showed also that the reaction had not yielded a single product.

In particular, the N,O-dimethylate product (22%), the O-monomethylate product (23%), the N,N,O-trimethylate product (18%), further O-methylated urethane (p- CH3OC6H4NHCO2CH3, 6%), and N,O-dimethylated urethane (8%) were identified.

From what is reported in the literature, it is reasonable to believe that the same lack of selectivity shown in example 14 can also be found if the initial substrate is constituted by aminobenzamides or aminobenzoic acids.

Therefore, it is now evident that the combination according to the present invention between organic carbonates [(III) or (IV)] and faujasites as catalysts allows to work at low temperatures while maintaining, and often even improving, the yield and most of all the selectivity of known equivalent processes.

The disclosures in Italian Patent Application No. PD2002A000325 from which this application claims priority are incorporated herein by reference.

## Claims

1. A process for the direct synthesis of mono-N-substituted anilines having the general formula (I) 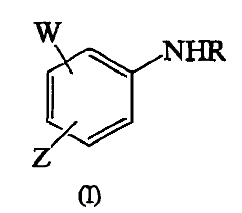 wherein
R indicates a linear or branched saturated carbon chain, preferably comprising 1 to 7 carbon atoms, an unsaturated carbon chain with the double carbon-carbon link also in the allyl position (2,3) with respect to the nitrogen atom of the amines (I), comprising 3 to 7 carbon atoms, or a benzyl group or a benzyl group substituted at the aromatic ring with methyl and ethyl radicals;
W is selected from the group consisting of -OH, -CH2OH, -COOH and -CONH₂ and can be ortho, meta or para with respect to the carbon atom to which the nitrogen atom is attached;
Z is selected from the group consisting of -H, -halogen, -alkyl, -alkoxy, - N02 and -CN;
said process comprising the step of reacting, in the presence of a solvent, a compound having the general formula (II): 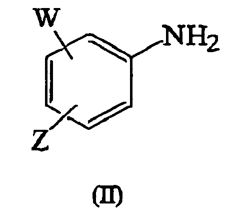 wherein W and Z are as defined above,
with an organic carbonate selected from the group consisting of compounds having the general formula (III) 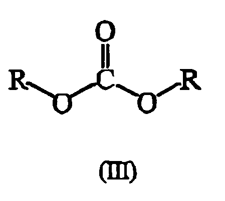 wherein R is as defined above,
and compounds of general formula (IV) 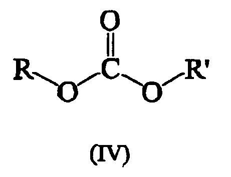 wherein R' is selected from the group consisting of CH₃(OCH₂CH₂)n- with n ≥ 2 and branched or linear alkyl radicals that have at least three carbon atoms;
in the presence of a faujasite selected from the group comprising X-faujasite exchanged with alkaline metals and Y- faujasite exchanged with alkaline metals.

2. The process according to claim 1, wherein the organic carbonate has the general formula (III).

3. The process according to claim 1, wherein the organic carbonate has the general formula (IV).

4. The process according to claims 1 to 3, wherein the radical R is selected from the group consisting of methyl, ethyl, allyl and benzyl.

5. The process according to claim 1, wherein said faujasite is present in a ratio between 1:10 and 3:1 with respect to the compound having the formula (I).

6. The process according to claim 5, wherein said faujasite is present in a ratio between 1:1.5 and 1:1 with respect to the compound having the formula (I).

7. The process according to any one of claims 1 to 6, wherein said faujasite is Y-faujasite exchanged with sodium.

8. The process according to claim 1, wherein said organic carbonate is dimethyl carbonate.

9. The process according to claim 1, wherein said organic carbonate is diethyl carbonate.

10. The process according to claim 1, wherein said organic carbonate is diallyl carbonate.

11. The process according to claim 1, wherein said organic carbonate is dibenzyl carbonate

12. The process according to claim 1, wherein said organic carbonate is 2-(2-methoxyethoxy)ethyl-methylcarbonate.

13. The process according to claim 1, wherein said organic carbonate is 2-(2-methoxyethoxy)ethyl-ethylcarbonate.

14. The process according to claim 1, wherein the organic carbonate is present in a ratio between 10:1 and 50:1 with respect to the compound having the formula (I).

15. The process according to claim 1, wherein said step is performed at a temperature between 70°C and 190°C.

16. The process according to claim 15, wherein said temperature is between 90°C and 150°C.

17. The process according to claim 8, wherein said step is performed at a temperature between 70°C and 90°C.

18. The process according to claim 9, wherein said step is performed at a temperature between 70°C and 130°C.

19. The process according to any one of claims 10 and 11, wherein said step is performed at a temperature between 130°C and 190°C.

20. The process according to claim 1, wherein said step is performed at atmospheric pressure.

21. The process according to claim 1, wherein said step is performed in an autoclave.

22. The process according to claim 1, wherein said step is performed in atmosphere that is modified by adding an inert gas selected from the group consisting of nitrogen and argon.

23. The process according to claim 1, wherein said solvent is selected from the group consisting of:
said organic carbonate,
a co-solvent, and
mixtures thereof.

24. The process according to claim 23, wherein said co-solvent is selected from the group consisting of 1,2-dimethoxyethane, triethylene glycol dimethyl ether, and mixtures thereof.

25. The process according to any one of claims 23 and 24, wherein said solvent is a mixture of said organic carbonate and of a co-solvent and said co-solvent is preferably present in a ratio comprised between 1:1 and 5:1 with respect to the organic carbonate.

26. The use of faujasites exchanged with alkaline metals to catalyse reactions for mono-N-substitutionof anilines having the general formula (I) as defined in claim 1, wherein said faujasites are used together with an organic carbonate selected from the group consisting of compounds having the general formula (III) and compounds having the general formula (IV), as defined in claim 1.

27. The use according to claim 26, wherein said faujasite is NaY-faujasite.

## Patentansprüche

1. Verfahren zur unmittelbaren Synthese von Mono-N-Substituierten Anilinen mit der allgemeinen Formel (I) 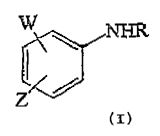 wobei
R bedeutet eine lineare oder verzweigte gesättigte Kohlenstoffkette, bevorzugt umfassend 1 bis 7 Kohlenstoffatome, eine ungesättigte Kohlenstoffkette mit der Kohlenstoff-Kohlenstoffdoppelbindung und in Bezug auf das Stickstoffatom des Amines (I) in der Allylstellung (2, 3), umfassend 3 bis 7 Kohlenstoffatome, oder eine Benzylgruppe oder eine Benzylgruppe, die am aromatischen Ring mit Methyl- und Ethylresten substituiert ist;
W ausgewählt ist aus der Gruppe, bestehend aus -OH, -CH₂OH, -COOH und -CONH₂ und in Ortho-, Meta- oder Parastellung stehen kann, bezogen auf das Kohlenstoffatom, an das das Stickstoffatom gebunden ist;
Z ausgewählt ist aus der Gruppe, bestehend aus -H, -Halogen, -Alkyl, -Alkoxy, -NO₂ und -CN;
wobei das Verfahren den Schritt umfasst:
umsetzen in Gegenwart eines Lösungsmittels einer Verbindung mit der allgemeinen Formel (II): 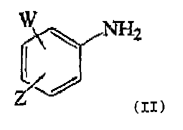 wobei W und Z die oben definierte Bedeutung besitzen,
mit einem organischen Carbonat, ausgewählt aus der Gruppe bestehend aus Verbindungen mit der allgemeinen Formel (III), 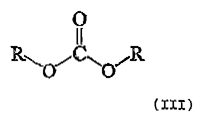 wobei R die oben definierte Bedeutung besitzt,
und Verbindungen der allgemeinen Formel (IV), 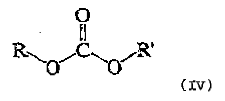 wobei R' ausgewählt ist aus der Gruppe, bestehend aus CH₃(OCH₂CH₂)ₙ- mit *n* ≥ 2 und verzweigten oder linearen Alkylresten, die wenigstens drei Kohlenstoffatome umfassen,
in der Gegenwart eines Faujasites, ausgewählt aus der Gruppe umfassend mit Alkalimetallen substituierte X-Faujasite und mit Alkalimetallen substituierte Y-Faujasite.

2. Verfahren nach Anspruch 1, wobei das organische Carbonat die allgemeine Formel (III) besitzt.

3. Verfahren nach Anspruch 1, wobei das organische Carbonat die allgemeine Formel (IV) besitzt.

4. Verfahren nach einem der Ansprüche 1 bis 3, wobei der Rest R ausgewählt ist aus der Gruppe, bestehend aus Methyl. Ethyl, Allyl und Benzyl.

5. Verfahren nach Anspruch 1, wobei das Faujasit in einem Verhältnis von 1:10 bis 3:1 bezogen auf die Verbindung der Formel (1) vorhanden ist.

6. Verfahren nach Anspruch 5, wobei das Faujasit in einem Verhältnis von 1:1,5 bis 1:1 bezogen auf die Verbindung mit der Formel (1) vorhanden ist,

7. Verfahren nach einem der Ansprüche 1 bis 6, wobei das Faujasit mit Natrium substituiertes Y-Faujasit ist.

8. Verfahren nach Anspruch 1, wobei das organische Carbonat Dimethylcarbonat ist.

9. Verfahren nach Anspruch 1, wobei das organische Carbonat Diethylcarbonat ist.

10. Verfahren nach Anspruch 1, wobei das organische Carbonat Diallylcarbonat ist.

11. Verfahren nach Anspruch 1, wobei das organische Carbonat Dibenzylcarbonat ist.

12. Verfahren nach Anspruch 1, wobei das organische Carbonat 2-(2-Methoxyethoxy)-ethyl-methylcarbonat ist.

13. Verfahren nach Anspruch 1, wobei das organische Carbonat 2-(2-Methoxyethoxy)-ethyl-ethylcarbonat ist.

14. Verfahren nach Anspruch 1, wobei das organische Carbonat in einem Verhältnis von 10:1 bis 50:1 bezogen auf die Verbindung mit der Formel (I) vorhanden ist.

15. Verfahren nach Anspruch 1, wobei der Schritt bei einer Temperatur von 70 °C bis 190 °C ausgeführt wird.

16. Verfahren nach Anspruch 15, wobei die Temperatur zwischen 90 °C und 160 °C ist.

17. Verfahren nach Anspruch 8, wobei der Schritt bei einer Temperatur von 70 °C bis 90 °C durchgeführt wird.

18. Verfahren nach Anspruch 9, wobei der Schritt bei einer Temperatur von 70 °C bis 130 °C durchgeführt wird.

19. Verfahren nach einem der Ansprüche 10 oder 11, wobei der Schritt bei einer Temperatur von 130 °C bis 190 °C durchgeführt wird.

20. Verfahren nach Anspruch 1, wobei der Schritt bei Atmosphärendruck durchgeführt wird.

21. Verfahren nach Anspruch 1, wobei der Schritt in einem Autoklaven durchgeführt wird.

22. Verfahren, nach Anspruch 1, wobei der Schritt in einer Atmosphäre durchgeführt wird, die verändert wurde durch Hinzufügen eines inerten Gases, ausgewählt aus der Gruppe bestehend aus Stickstoff und Argon.

23. Verfahren nach Anspruch 1, wobei das Lösungsmittel ausgewählt ist aus der Gruppe, bestehend aus:
dem organischen Carbonat,
einem Cosolvent, und
deren Mischungen.

24. Verfahren nach Anspruch 23, wobei das Cosolvent ausgewählt ist aus der Gruppe, bestehend aus 1,2-Dimethoxyethan, Triethylenglykoldimethylether und deren Mischungen.

25. Verfahren nach einem der Ansprüche 23 oder 24, wobei das Lösungsmittel eine Mischung aus dem organischen Carbonat und einem Cosolvent ist und das Cosolvent bevorzugt in einem Verhältnis von 1:1 bis 5:1 bezogen auf das organische Carbonat enthalten ist.

26. Verwendung von mit Alkalimetallen substituierten Faujasiten zur Katalysse von Reaktionen zur Mono-N-Substiution von Anilinen der allgemeinen Formel (I) wie in Anspruch 1 definiert, wobei die Faujasite gemeinsam verwendet werden mit einem organischen Carbonat ausgewählt aus der Gruppe bestehend aus Verbindungen der allgemeinen Formel (111) und Verbindungen der allgemeinen Formel (IV), wie in Anspruch 1 definiert.

27. Verwendung nach Anspruch 26, wobei das Faujasit ein Na-Y-Faujasft ist.

## Revendications

1. Procédé de synthèse directe d'anilines mono-N-substituées de formule générale (1) 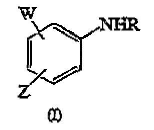 dans laquelle
- R désigne une chaîne carbonée saturée linéaire ou ramifiée, comprenant de préférence 1 à 7 atomes de carbone, une chaîne carbonée insaturée dont la double liaison carbone-carbone se trouve également dans la position =allyle (2,3) par rapport à l'atome d'azote des amines (I), comprenant 3 à 7 atomes de carbone, ou un groupe benzyle ou un groupe benzyle substitué au niveau du noyau aromatique par des radicaux méthyle et éthyle;
- W est choisi dans le groupe constitué par -OH, -CH₂OH, -COOH et -CONH₂ et peut être ortho, meta ou para par rapport à l'atome de carbone auquel l'atome d'azote est fixé;
- Z est choisi dans le groupe constitué par un groupe -H, halogéno, alkyle, alcoxyle, - NO₂ et -CN ;
ledit procédé comprenant l'étape consistant à faire réagir, en présence d'un solvant, un composé de formule générale (II) : 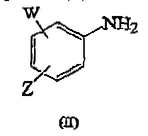 dans laquelle W et Z sont tels que définis ci-dessus,
avec un carbonate organique choisi dans le groupe constitué par les composés de formule générale (III) 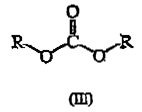 dans laquelle R est tel que défini ci-dessus,
et les composés de formule générale (IV) 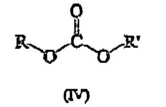 dans laquelle R' est choisi dans le groupe constitué par CH₃(OCH₂CH₂)n- avec n≥2 et les radicaux alkyle linéaires ou ramifiés qui comprennent au moins trois atomes de carbone ;
en présence d'une faujasite choisie dans le groupe comprenant la faujasite X échangée avec des métaux alcalins et la faujasite Y échangée avec des métaux alcalins.

2. Procédé selon la revendication 1, dans lequel le carbonate organique a la formule générale (III).

3. Procédé selon la revendication 1, dans lequel le carbonate organique a la formule générale (IV).

4. Procédé selon les revendications 1 à 3, dans lequel le radical R est choisi dans le groupe constitué par les groupes méthyle, éthyle, allyle et benzyle.

5. Procédé selon la revendication 1, dans lequel ladite faujasite est présente selon un rapport compris entre 1:10 et 3:1 par rapport au composé de formule (I).

6. Procédé selon la revendication 5, dans lequel ladite faujasite est présente selon un rapport compris entre 1;1,5 et 1:1 par rapport au composé de formule (I).

7. Procédé selon l'une quelconque des revendications 1 à 6, dans lequel ladite faujasite est la faujasite Y échangée avec le sodium.

8. Procédé selon la revendication 1, dans lequel ledit carbonate organique est le carbonate de diméthyle

9. Procédé selon la revendication 1, dans lequel ledit carbonate organique est le carbonate de diéthyle.

10. Procédé selon la revendication 1, dans lequel ledit carbonate organique est le carbonate de diallyle.

11. Procédé selon la revendication 1, dans lequel ledit carbonate organique est le carbonate de dibenzyle.

12. Procédé selon la revendication 1, dans lequel ledit carbonate organique est le méthylcarbonate de 2-(2-méthoxyéthoxy)éthyle.

13. Procédé selon la revendication 1, dans lequel ledit carbonate organique est l'éthylcarbonate de 2-(2-méthoxyéthoxy)éthyle.

14. Procédé selon la revendication 1, dans lequel le carbonate organique est présent selon un rapport compris entre 10:1 et 50:1 par rapport au composé de formule (1).

15. Procédé selon la revendication 1, dans lequel ladite étape est réalisée à une température comprise entre 70°C et 190°C.

16. Procédé selon la revendication 15, dans lequel ladite température est comprise entre 90°C et 150°C.

17. Procédé selon la revendication 8, dans lequel ladite étape est réalisée à une température comprise entre 70°C et 90°C.

18. Procédé selon la revendication 9, dans lequel ladite étape est réalisée à une température comprise entre 70°C et 130°C.

19. Procédé selon l'une quelconque des revendications 10 et 11, dans lequel ladite étape est réalisée à une température comprise entre 130°C et 190°C.

20. Procédé selon la revendication 1, dans lequel ladite étape est réalisée sous pression atmosphérique.

21. Procédé selon la revendication 1, dans lequel ladite étape est réalisée dans un autoclave.

22. Procédé selon la revendication 1, dans lequel ladite étape est réalisée sous une atmosphère qui est modifiée par l'addition d'un gaz inerte choisi dans le groupe constitué par l'azote et l'argon.

23. Procédé selon la revendication 1, dans lequel ledit solvant est choisi dans le groupe constitué, par :
- ledit carbonate organique,
- un co-solvant, et
- leurs mélanges.

24. Procédé selon la revendication 23, dans lequel ledit co-solvant est choisi dans le groupe constitué par le 1,2-diméthoxyéthane, l'éther diméthylique, de triéthylèneglycol et leurs mélanges.

25. Procédé selon l'une quelconque des revendications 23 et 24, dans lequel ledit solvant est un mélange dudit carbonate organique et d'un co-solvant et ledit co-solvant est de préférence présent selon un rapport compris entre 1:1 et 5:1 par rapport au carbonate organique.

26. Utilisation de faujasites échangées avec des métaux alcalins pour catalyser des réactions de mono-N-substitution d'anilines de formule générale (I) telle que définie dans la revendication 1, dans laquelle lesdites faujasites sont utilisées conjointement avec un carbonate organique choisi dans le groupe constitué par les composés de formule générale (III) et les composés de formule générale (IV) telles que définies dans la revendication 1.

27. Utilisation selon la revendication 26, dans laquelle ladite faujasite est la Faujasite NaY.
